(19) Europäisches Patentamt
European Patent Office
Office européen des brevets

(11) Veröffentlichungsnummer: **0 019 742**
**A2**

# (12) EUROPÄISCHE PATENTANMELDUNG

(21) Anmeldenummer: **80102375.5**

(22) Anmeldetag: **02.05.80**

(51) Int. Cl.³: **C 07 D 271/08**, C 07 D 285/10, C 07 D 249/04, A 01 N 43/64, A 01 N 43/82

(30) Priorität: **12.05.79 DE 2919293**

(43) Veröffentlichungstag der Anmeldung: **10.12.80 Patentblatt 80/25**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Stetter, Jörg, Dr., Gellertweg 4, D-5600 Wuppertal 1 (DE)**
Erfinder: **Ditgens, Klaus, Dr., Claudiusweg 7, D-5600 Wuppertal 1 (DE)**
Erfinder: **Thomas, Rudolf, Dr., Wilkhausstrasse 129, D-5600 Wuppertal (DE)**
Erfinder: **Eue, Ludwig, Dr., Paul-Klee-Strasse 36, D-5090 Leverkusen (DE)**
Erfinder: **Schmidt, Robert Rudolf, Hahnenweg 5, D-5000 Köln (DE)**

## (54) N-(2,5-Diazolyl)alkyl-halogenacetanilide, Verfahren zu ihrer Herstellung sowie ihre Verwendung als Herbizide.

(57) Neue N-(2,5-Diazolyl)-alkyl-halogenacetanilide der Formel

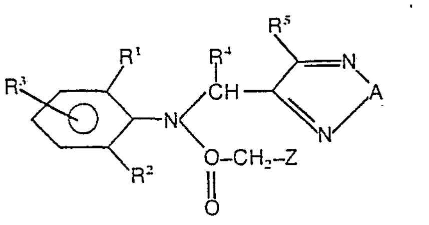

(I)

in welcher

A für Sauerstoff, Schwefel oder die Gruppierung >NH steht, worin

R für Alkyl steht,

$R^1$ für Wasserstoff, Alkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wasserstoff oder Alkyl steht,

$R^5$ für Wasserstoff, Alkyl, Alkoxy, Alkylthio, Dialkylamino, Halogen, Cyano oder Alkoxycarbonyl steht und

Z für Halogen steht,

sowie deren Säureadditions-Salze und Metallsalz-Komplexe, mehrere Verfahren zu ihrer Herstellung sowie ihre Verwendung als Herbizide.

Neue N-(2,5-Diazolyl)-alkyl-aniline der Formel

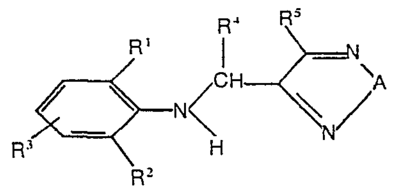

(II)

in welcher $R^1$, $R^2$, $R^3$, $R^4$, $R^5$ und A die oben angegebene Bedeutung haben, und ein Verfahren zur Herstellung dieser Stoffe.

EP 0 019 742 A2

0019742

BAYER AKTIENGESELLSCHAFT 5090 Leverkusen, Bayerwerk
Zentralbereich
Patente, Marken und Lizenzen Dü-kl-Kü
Ib

N-(2,5-Diazolyl)alkyl-halogenacetanilide, Verfahren zu ihrer Herstellung sowie ihre Verwendung als Herbizide

Die vorliegende Erfindung betrifft neue N-(2,5-Diazolyl) alkyl-halogenacetanilide, mehrere Verfahren zu ihrer Herstellung sowie ihre Verwendung als Herbizide.

Es ist bereits bekannt geworden, daß man 2,6-Diethyl-N-methoxymethyl-chloracetanilid zur Unkrautbekämpfung verwenden kann (vergleiche US-PS 3 442 945).
Diese Verbindung ist jedoch nicht immer ausreichend wirksam und in ihrer Selektivität nicht immer ganz befriedigend.

Es wurden nun neue N-(2,5-Diazolyl)alkyl-halogenacetanilide der Formel

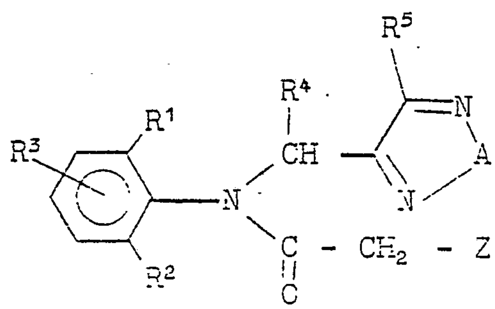

(I)

in welcher

A für Sauerstoff, Schwefel oder die Gruppierung >NR steht, worin

R für Alkyl steht,

$R^1$ für Wasserstoff, Alkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wasserstoff oder Alkyl steht,

$R^5$ für Wasserstoff, Alkyl, Alkoxy, Alkylthio, Dialkylamino, Halogen, Cyano oder Alkoxycarbonyl steht und

Z für Halogen steht,

sowie deren Säureadditions-Salze und Metallsalz-Komplexe gefunden.

Weiterhin wurde gefunden, daß man die N-(2,5-Diazolyl)-alkyl-halogenacetanilide der Formel (I) sowie deren Säureadditions-Salze und Metallsalz-Komplexe erhält, wenn man

a) N-(2,5-Diazolyl)alkyl-aniline der Formel

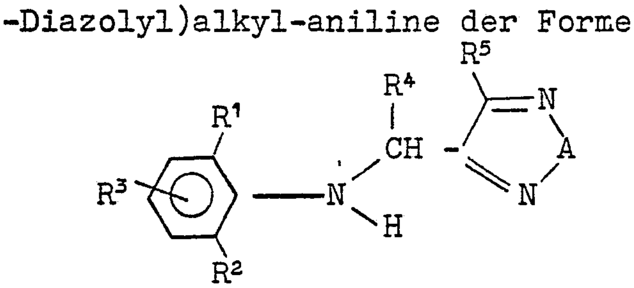

(II)

in welcher

A, $R^1$, $R^2$, $R^3$,
$R^4$ und $R^5$ die oben angegebene Bedeutung haben,

mit Halogenessigsäurechloriden oder -bromiden bzw. -anhydriden der Formeln

$$Z - CH_2 - CO - Cl(Br) \qquad (IIIa)$$

bzw.

$$(Z - CH_2 - CO)_2O \qquad (IIIb)$$

in welchen

Z die oben angegebene Bedeutung hat,

in Gegenwart eines Verdünnungsmittels und gegebenenfalls in Gegenwart eines Säurebindemittels umsetzt, oder

b) Halogenacetanilide der Formel

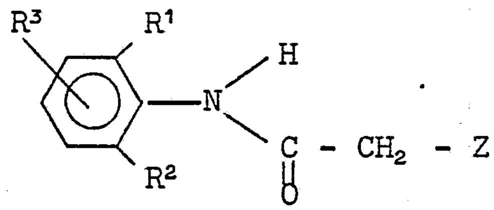

(IV)

in welcher

$R^1$, $R^2$, $R^3$, und Z die oben angegebene Bedeutung haben,

mit Diazolylalkyl-Derivaten der Formel

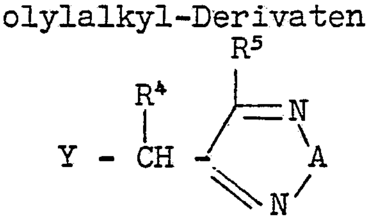

(V)

in welcher

A, $R^4$ und $R^5$ die oben angegebene Bedeutung haben und

Y für Halogen, den Mesyl- oder Tosyl-Rest steht,

in Gegenwart eines Säurebinders und gegebenenfalls in Gegenwart eines organischen Verdünnungsmittels, oder in einem wässrig-organischen Zweiphasensystem in Gegenwart eines Phasentransferkatalysators umsetzt,

und gegebenenfalls anschließend eine Säure oder ein Metallsalz addiert.

Außerdem wurde gefunden, daß die neuen N-(2,5-Diazolyl)-alkyl-halogenacetanilide der Formel (I) sowie deren Säureadditions-Salze und Metallsalz-Komplexe starke herbizide, insbesondere auch selektiv-herbizide Eigenschaften aufweisen.

Ueberraschenderweise zeigen die erfindungsgemäßen N-(2,5-Diazolyl)alkyl-halogenacetanilide bei sehr guter Unkrautwirkung bessere Möglichkeiten zum Einsatz als selektive Unkrautbekämpfungsmittel in wichtigen Kulturpflanzen als das aus dem Stand der Technik bekannte 2,6-Diethyl-N-methoxymethyl-chloracetanilid, welches ein hochaktiver Wirkstoff gleicher Wirkungsart ist. Die erfindungsgemäßen Stoffe stellen somit eine wertvolle Bereicherung der Technik dar.

Die erfindungsgemäßen N-(2,5-Diazolyl)alkyl-halogenacetanilide sind durch die Formel (I) allgemein definiert. In der Formel (I) steht A vorzugsweise für Sauerstoff, Schwefel oder die Gruppierung >NR, wobei R vorzugsweise für geradkettiges oder verzweigtes Alkyl mit 1 bis 4 Kohlenstoffatomen steht. $R^1$ steht vorzugsweise für Wasserstoff und geradkettiges oder verzweigtes Alkyl und Alkoxy mit jeweils 1 bis 4 Kohlenstoffatomen. $R^2$ steht vorzugsweise für Wasserstoff, geradkettiges oder verzweigtes Alkyl oder Alkoxy mit jeweils 1 bis 4 Kohlenstoffatomen, sowie für Fluor, Chlor und Brom. $R^3$ steht ebenfalls vorzugsweise für Wasserstoff,

geradkettiges oder verzweigtes Alkyl und Alkoxy mit jeweils 1 bis 4 Kohlenstoffatomen, sowie für die Halogene Fluor, Chlor und Brom. $\underline{R}^4$ steht vorzugsweise für Wasserstoff oder Alkyl mit 1 bis 4 Kohlenstoffatomen. $\underline{R}^5$ steht vorzugsweise für Wasserstoff, für geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, Alkoxycarbonyl mit 1 bis 4 Kohlenstoffatomen in der Alkoxygruppe, weiterhin für Dialkylamino mit 1 bis 4 Kohlenstoffatomen in jedem Alkylteil, für Fluor, Chlor und Brom, sowie für Cyano. Z steht vorzugsweise für Chlor, Brom und Jod.

Ganz besonders bevorzugt sind diejenigen N-( 2,5-Diazolyl)alkyl-halogenacetanilide der Formel (I), in denen A für Sauerstoff, Schwefel oder die Gruppierung >NR steht, wobei R für Methyl, Ethyl, Propyl oder Butyl steht; $\underline{R}^1$ für Wasserstoff, Methyl, Ethyl, Isopropyl, sek.-Butyl, tert.-Butyl, Methoxy, Ethoxy oder Isopropoxy steht; $\underline{R}^2$ für Wasserstoff, Methyl, Ethyl, Isopropyl, sek.-Butyl, tert.-Butyl, Methoxy, Ethoxy, Isopropoxy, Chlor oder Brom steht; $\underline{R}^3$ für Wasserstoff, Methyl, Ethyl, Isopropyl, sek.-Butyl, tert.-Butyl, Methoxy, Ethoxy, Isopropoxy, Chlor oder Brom steht; $\underline{R}^4$ für Wasserstoff oder Methyl steht. $\underline{R}^5$ für Wasserstoff, Methyl, Ethyl, n-Propyl, Isopropyl, n-Butyl, sek.-Butyl, tert.-Butyl, Methoxy, Ethoxy, Isopropoxy, Methylthio, Ethylthio, Isopropylthio, Methoxycarbonyl, Ethoxycarbonyl, Fluor, Chlor, Brom, Cyano, Dimethylamino, Diethylamino und Ethylmethylamino steht; und Z für Chlor oder Brom steht.

Im einzelnen seien außer den bei den Herstellungsbeispielen genannten Verbindungen die folgenden Verbindungen der allgemeinen Formel (I) genannt:

Tabelle 1



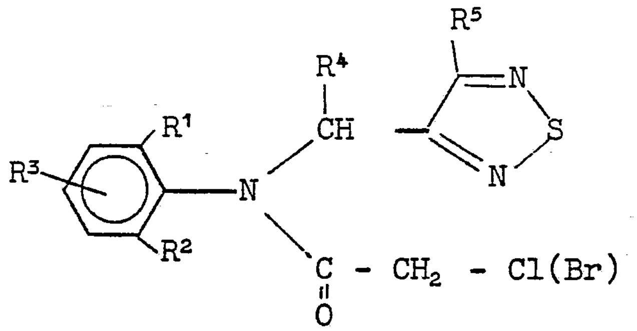

(Ia)

| $R^1$ | $R^2$ | $R^3$ | $R^4$ | $R^5$ |
|---|---|---|---|---|
| $C(CH_3)_3$ | H | H | H | $CH_3$ |
| $CH_3$ | H | 3-$CH_3$ | H | $CH_3$ |
| $CH_3$ | H | 5-$CH_3$ | H | $CH_3$ |
| $CH_3$ | $OCH_3$ | H | H | $CH_3$ |
| $OCH_3$ | $OCH_3$ | H | H | $CH_3$ |
| $CH_3$ | Cl | H | H | $CH_3$ |
| $C(CH_3)_3$ | Cl | H | H | $CH_3$ |
| $C_2H_5$ | $C_2H_5$ | H | H | H |
| $CH_3$ | $C_2H_5$ | H | H | H |
| $CH_3$ | $CH_3$ | H | H | H |
| $C(CH_3)_3$ | H | H | H | H |
| $CH_3$ | H | 3-$CH_3$ | H | H |
| $CH_3$ | H | 5-$CH_3$ | H | H |
| $CH_3$ | $OCH_3$ | H | H | H |
| $OCH_3$ | $OCH_3$ | H | H | H |
| $CH_3$ | Cl | H | H | H |
| $C(CH_3)_3$ | Cl | H | H | H |
| $C_2H_5$ | $C_2H_5$ | H | $CH_3$ | $CH_3$ |
| $CH_3$ | $C_2H_5$ | H | $CH_3$ | $CH_3$ |
| $CH_3$ | $CH_3$ | H | $CH_3$ | $CH_3$ |
| $C(CH_3)_3$ | H | H | $CH_3$ | $CH_3$ |

Tabelle 1 (Fortsetzung)

| $R^1$ | $R^2$ | $R^3$ | $R^4$ | $R^5$ |
|---|---|---|---|---|
| $CH_3$ | H | 3-$CH_3$ | $CH_3$ | $CH_3$ |
| $CH_3$ | H | 5-$CH_3$ | $CH_3$ | $CH_3$ |
| $CH_3$ | $OCH_3$ | H | $CH_3$ | $CH_3$ |
| $OCH_3$ | $OCH_3$ | H | $CH_3$ | $CH_3$ |
| $CH_3$ | Cl | H | $CH_3$ | $CH_3$ |
| $C(CH_3)_3$ | $CH_3$ | H | $CH_3$ | $CH_3$ |
| $C_2H_5$ | $C_2H_5$ | H | $CH_3$ | H |
| $CH_3$ | $C_2H_5$ | H | $CH_3$ | H |
| $CH_3$ | $CH_3$ | H | $CH_3$ | H |
| $C_2H_5$ | $C_2H_5$ | H | H | $C_2H_5$ |
| $CH_3$ | $C_2H_5$ | H | H | $C_2H_5$ |
| $CH_3$ | $CH_3$ | H | H | $C_2H_5$ |
| $C_2H_5$ | $C_2H_5$ | H | H | Cl |
| $CH_3$ | $C_2H_5$ | H | H | Cl |
| $CH_3$ | $CH_3$ | H | H | Cl |
| $C_2H_5$ | $C_2H_5$ | H | H | $OCH_3$ |
| $CH_3$ | $C_2H_5$ | H | H | $OCH_3$ |
| $CH_3$ | $CH_3$ | H | H | $OCH_3$ |
| $C_2H_5$ | $C_2H_5$ | H | H | $SCH_3$ |
| $CH_3$ | $C_2H_5$ | H | H | $SCH_3$ |
| $CH_3$ | $CH_3$ | H | H | $SCH_3$ |

Tabelle 2

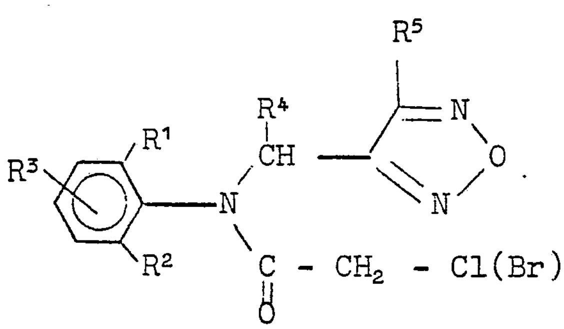

(Ib)

Tabelle 2 (Fortsetzung)

| $R^1$ | $R^2$ | $R^3$ | $R^4$ | $R^5$ |
|---|---|---|---|---|
| $C(CH_3)_3$ | H | H | H | $CH_3$ |
| $CH_3$ | H | 3-$CH_3$ | H | $CH_3$ |
| $CH_3$ | H | 5-$CH_3$ | H | $CH_3$ |
| $CH_3$ | $OCH_3$ | H | H | $CH_3$ |
| $OCH_3$ | $OCH_3$ | H | H | $CH_3$ |
| $CH_3$ | Cl | H | H | $CH_3$ |
| $C(CH_3)_3$ | Cl | H | H | $CH_3$ |
| $C(CH_3)_3$ | H | H | $CH_3$ | $CH_3$ |
| $CH_3$ | H | 3-$CH_3$ | $CH_3$ | $CH_3$ |
| $CH_3$ | H | 5-$CH_3$ | $CH_3$ | $CH_3$ |
| $CH_3$ | $OCH_3$ | H | $CH_3$ | $CH_3$ |
| $OCH_3$ | $OCH_3$ | H | $CH_3$ | $CH_3$ |
| $CH_3$ | Cl | H | $CH_3$ | $CH_3$ |
| $C(CH_3)_3$ | Cl | H | $CH_3$ | $CH_3$ |
| $C_2H_5$ | $C_2H_5$ | H | $CH_3$ | H |
| $CH_3$ | $C_2H_5$ | H | $CH_3$ | H |
| $CH_3$ | $CH_3$ | H | $CH_3$ | H |
| $C_2H_5$ | $C_2H_5$ | H | H | $C_2H_5$ |
| $CH_3$ | $C_2H_5$ | H | H | $C_2H_5$ |
| $CH_3$ | $CH_3$ | H | H | $C_2H_5$ |
| $C_2H_5$ | $C_2H_5$ | H | H | Cl |
| $CH_3$ | $C_2H_5$ | H | H | Cl |
| $CH_3$ | $CH_3$ | H | H | Cl |

Tabelle 2 (Fortsetzung)

| $R^1$ | $R^2$ | $R^3$ | $R^4$ | $R^5$ |
|---|---|---|---|---|
| $C_2H_5$ | $C_2H_5$ | H | H | $OCH_3$ |
| $CH_3$ | $C_2H_5$ | H | H | $OCH_3$ |
| $CH_3$ | $CH_3$ | H | H | $OCH_3$ |
| $C_2H_5$ | $C_2H_5$ | H | H | $SCH_3$ |
| $CH_3$ | $C_2H_5$ | H | H | $SCH_3$ |
| $CH_3$ | $CH_3$ | H | H | $SCH_3$ |

Tabelle 3

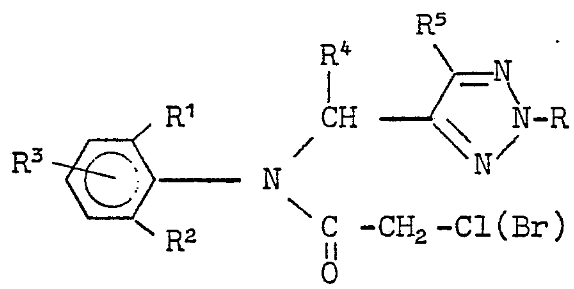

(Ic)

| $R^1$ | $R^2$ | $R^3$ | $R^4$ | $R^5$ | R |
|---|---|---|---|---|---|
| $CH_3$ | $CH_3$ | H | H | H | $CH_3$ |
| $CH_3$ | $C_2H_5$ | H | H | H | $CH_3$ |
| $C_2H_5$ | $C_2H_5$ | H | H | H | $CH_3$ |
| $CH_3$ | $CH_3$ | H | $CH_3$ | H | $CH_3$ |
| $CH_3$ | $C_2H_5$ | H | $CH_3$ | H | $CH_3$ |
| $C_2H_5$ | $C_2H_5$ | H | $CH_3$ | H | $CH_3$ |
| $CH_3$ | $CH_3$ | H | H | $CH_3$ | $CH_3$ |
| $CH_3$ | $C_2H_5$ | H | H | $CH_3$ | $CH_3$ |
| $C_2H_5$ | $C_2H_5$ | H | H | $CH_3$ | $CH_3$ |

Tabelle 3 (Fortsetzung)

| $R^1$ | $R^2$ | $R^3$ | $R^4$ | $R^5$ | R |
|---|---|---|---|---|---|
| $CH_3$ | $CH_3$ | H | $CH_3$ | $CH_3$ | $CH_3$ |
| $CH_3$ | $C_2H_5$ | H | $CH_3$ | $CH_3$ | $CH_3$ |
| $C_2H_5$ | $C_2H_5$ | H | $CH_3$ | $CH_3$ | $CH_3$ |
| $CH_3$ | $CH_3$ | H | H | $OCH_3$ | $CH_3$ |
| $CH_3$ | $C_2H_5$ | H | H | $OCH_3$ | $CH_3$ |
| $C_2H_5$ | $C_2H_5$ | H | H | $OCH_3$ | $CH_3$ |
| $CH_3$ | $CH_3$ | H | H | $SCH_3$ | $CH_3$ |
| $CH_3$ | $C_2H_5$ | H | H | $SCH_3$ | $CH_3$ |
| $C_2H_5$ | $C_2H_5$ | H | H | $SCH_3$ | $CH_3$ |

Bevorzugte erfindungsgemäße Verbindungen sind auch Additionsprodukte aus Säuren und denjenigen N-2,5-Diazolyl-alkyl-halo-enacetaniliden der Formel (I), in denen A, $R^1$, $R^2$, $R^3$, $R^4$, $R^5$ und Z die Bedeutungen haben, die bereits vorzugsweise für diese Reste genannt wurden. Zu den Säuren, die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppen des Periodensystems und denjenigen N-(2,5-Diazolyl)alkyl-halogenacetaniliden der Formel (I), in denen A, $R^1$, $R^2$, $R^3$, $R^4$, $R^5$ und Z die Bedeutungen haben, die bereits vorzugsweise für diese Reste genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. Chlorwasserstoffsäure und Bromwasserstoffsäure, ferner Phosphorsäure, Salpetersäure und Schwefelsäure.

Verwendet man 2-Ethyl-6-methyl-N-(4-methyl-1,2,5-thiadiazol-3-yl-methyl)-anilin und Chloracetylchlorid als Ausgangsstoffe, so kann der Reaktionsablauf durch das folgende Formelschema wiedergegeben werden (Verfahrensvariante a):

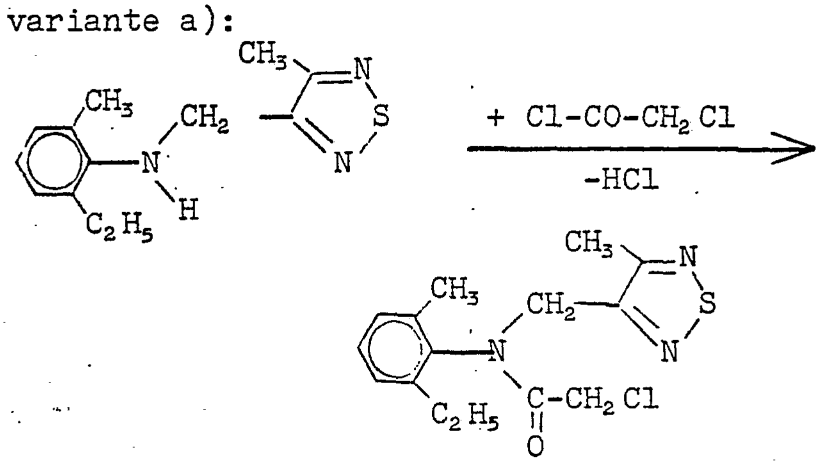

Verwendet man 2,6-Dimethyl-chloracetanilid und 3-Brommethyl-4-methyl-1,2,5-oxadiazol als Ausgangsstoffe, so kann der Reaktionsablauf durch das folgende Formelschema wiedergegeben werden (Verfahrensvariante b):

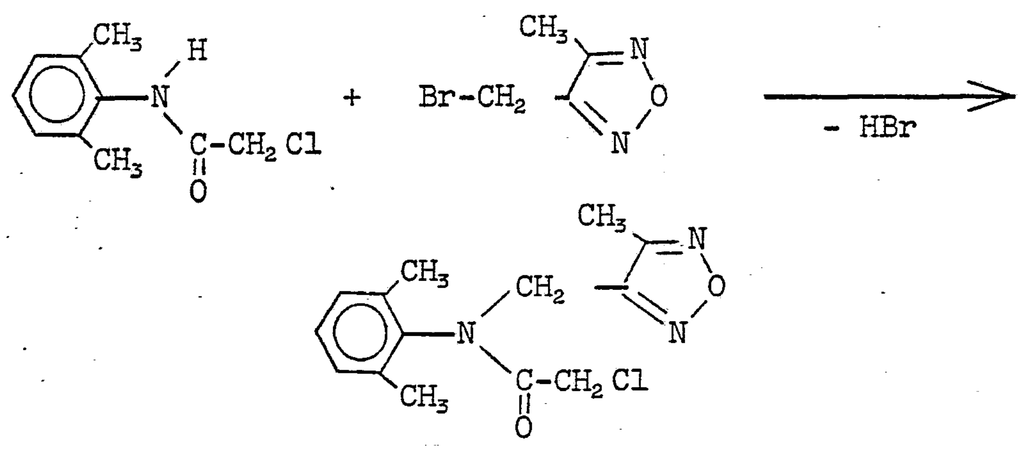


Die bei der Durchführung des erfindungsgemäßen Verfahrens (a) als Ausgangsstoffe benötigten N-(2,5-Diazolyl)alkyl-aniline sind durch die Formel (II) allgemein definiert. In dieser Formel stehen A, $R^1$, $R^2$, $R^3$, $R^4$ und $R^5$ vorzugsweise für diejenigen Reste, die bereits im Zusammenhang mit der Beschreibung der erfindungsgemäßen Stoffe der Formel (I) vorzugsweise für diese Reste genannt wurden.

Die N-(2,5-Diazolyl)alkyl-aniline der Formel (II) sind noch nicht bekannt. Man erhält sie, wenn man Aniline der Formel

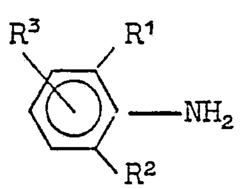
(VI)

in welcher

$R^1$, $R^2$ und $R^3$ die oben angegebene Bedeutung haben,

mit Diazolylalkyl-Derivaten der Formel

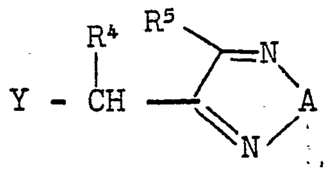

(V)

in welcher

A, $R^4$, $R^5$ und Y die oben angegebene Bedeutung haben,

in Gegenwart eines Säurebinders und gegebenenfalls in Gegenwart eines Verdünnungsmittels umsetzt.

Die bei der Herstellung der N-(2,5-Diazolyl)alkyl-aniline der Formel (II) als Ausgangsstoffe benötigten Aniline der Formel (VI) sind allgemein bekannte Verbindungen der organischen Chemie. Als Beispiele seien genannt:

Anilin; 2-Methylanilin,; 2-Ethylanilin; 2-Isopropylanilin; 2-sek.-Butylanilin; 2-tert.-Butylanilin; 2,6-Dimethylanilin; 2,3-Dimethylanilin; 2,5-Dimethylanilin; 3,5-Dimethylanilin; 2,6-Diethylanilin; 2-Ethyl-6-methylanilin; 2,3,4-Trimethylanilin; 2,4,6-Trimethylanilin; 2,4,5-Trimethylanilin; 2-Ethyl-4,6-dimethylanilin; 2,6-Diethyl-4-methylanilin; 2,6-Diisopropyl-4-methylanilin; 2,3,5-Trimethylanilin; 2,3,6-Trimethylanilin; 2-Methyl-6-chloranilin; 2-tert.-Butyl-6-chloranilin; 2-Methoxy-6-methylanilin; 2,6-Dimethoxyanilin; 2-Methoxy-6-ethoxyanilin; 2,6-Diethoxyanilin.

Bei der Herstellung der N-(2,5-Diazolyl)alkyl-aniline der Formel (II) können als Säurebinder alle üblichen Säureakzeptoren verwendet werden. Vorzugsweise in Betracht kommen Alkalicarbonate wie Kalium- oder Natriumcarbonat.

Als Verdünnungsmittel können bei der Herstellung der N-(2,5-Diazolyl)alkyl-aniline der Formel (II) alle üblichen inerten organischen Lösungsmittel eingesetzt werden. Vorzugsweise in Betracht kommen Dimethylformamid und Toluol.

Die Reaktionstemperaturen können bei der Herstellung der N-(2,5-Diazolyl)alkyl-aniline der Formel (II) nach dem obigen Verfahren innerhalb eines größeren Bereiches variiert werden. Im allgemeinen arbeitet man zwischen 0°C und 180°C, vorzugsweise zwischen 20°C und 160°C.

Bei der Herstellung der N-(2,5-Diazolyl)alkyl-aniline der Formel (II) nach dem obigen Verfahren setzt man die Aniline der Formel (VI) und die Diazolylalkyl-Derivate der Formel (V) im allgemeinen in äquimolaren Mengen ein. Es ist jedoch auch möglich, eine der Komponenten,vorzugsweise das Anilin der Formel (VI), in einem Ueberschuß einzusetzen.- Die Aufarbeitung und Isolierung der Reaktionsprodukte erfolgt nach üblichen Methoden (vgl. auch die Herstellungsbeispiele).

Die außerdem für das erfindungsgemäße Verfahren (a) als Ausgangsstoffe zu verwendenden Halogenessigsäurechloride und -bromide bzw. -anhydride sind durch die Formeln (IIIa) und (IIIb) allgemein definiert. In diesen Formeln steht Z vorzugsweise für Chlor, Brom und Jod.

Die Halogenessigsäurechloride und -bromide bzw. -anhydride der Formeln (IIIa) und (IIIb) sind allgemein bekannte Verbindungen der organischen Chemie. Als Beispiele seien genannt: Chloracetylchlorid, Bromacetylchlorid, Jodacetylchlorid und die entsprechenden Bromide sowie Anhydride.

Die bei der Durchführung des erfindungsgemäßen Verfahrens (b) als Ausgangsstoffe benötigten Halogenacetanilide sind durch die Formel (IV) allgemein definiert. In dieser Formel stehen $\underline{R}^1$, $\underline{R}^2$, $\underline{R}^3$ und $\underline{Z}$ vorzugsweise für diejenigen Reste, die bereits im Zusammenhang mit der Beschreibung der erfindungsgemäßen Stoffe der Formel (I) vorzugsweise für diese Reste genannt wurden.

Die Halogenacetanilide der Formel (IV) sind allgemein bekannt bzw. können sie in allgemein bekannter Art und Weise erhalten werden, indem man entsprechende Aniline mit einem Halogenessigsäurechlorid oder -bromid bzw. -anhydrid der Formeln (IIIa) bzw. (IIIb) in Gegenwart eines inerten organischen Lösungsmittels, wie beispielsweise Toluol oder Dimethylformamid, gegebenenfalls in Gegenwart eines Säurebindemittels, wie z.B. Kaliumcarbonat oder Triethylamin, bei Temperaturen zwischen 0°C und 100°C umsetzt (vergleiche auch die Herstellungsbeispiele). Als Beispiele seien die Chlor- und Bromacetanilide der oben angegebenen Aniline der Formel (VI) genannt.

Die außerdem für das erfindungsgemäße Verfahren (b) als Ausgangsstoffe zu verwendenden Diazolylalkyl-Derivate sind durch die Formel (V) allgemein definiert. In dieser Formel stehen $\underline{A}$, $\underline{R}^4$ und $\underline{R}^5$ vorzugsweise für diejenigen Reste, die bereits im Zusammenhang mit der Beschreibung der erfindungsgemäßen Stoffe der Formel (I) vorzugsweise für diese Reste genannt wurden. $\underline{Y}$ steht vorzugsweise für Chlor, Brom, den Mesyl- und Tosylrest.

Die Diazolyl-Derivate der Formel (V) sind teilweise bekannt (vergleiche J.Heterocyclic Chem. 4, 445-46 (1967)), bzw. können in allgemein bekannter Art und Weise erhalten werden, indem man z.B. bei Diazolyl-Derivaten der Formel

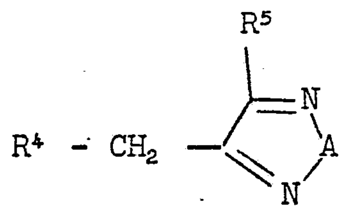

(VII)

in welcher

A, $R^4$ und $R^5$ die oben angegebene Bedeutung haben,

das aktive Wasserstoffatom in üblicher Weise gegen Halogen austauscht, wie z.B. durch Umsetzung mit N-Bromsuccinimid (vergleiche auch die Herstellungsbeispiele).

Die Diazolyl-Derivate der Formel (V) können auch erhalten werden, wenn man 3-Carbonsäureester-Derivate der Diazolyl-Derivate der Formel (VII) zu den entsprechenden 3-Hydroxymethyl-diazolyl-Derivaten reduziert und diese mit einem Halogenierungsmittel, wie beispielsweise Thionylchlorid oder Phosphortribromid, oder mit einem Sulfonylierungsmittel, wie Mesylchlorid oder Tosylchlorid, gegebenenfalls in Gegenwart eines inerten organischen Lösungsmittels, bei Temperaturen zwischen -20°C und +50°C umsetzt.

Die Diazolyl-Derivate der Formel (VII) sind bekannt (vergleiche u.a. Journal of Organic Chemistry, 32, 2823ff(1967)); bzw. können nach üblichen Heterocyclensynthesen erhalten werden.

Als Verdünnungsmittel kommen für die erfindungsgemäße Umsetzung gemäß Verfahrensvariante (a) vorzugsweise inerte organische Lösungsmittel infrage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Das erfindungsgemäße Verfahren (a) kann gegebenenfalls in Gegenwart von Säurebindern (z. B. Halogenwasserstoff-Akzeptoren) durchgeführt werden. Als solche können alle üblichen Säurebindemittel verwendet werden. Hierzu gehören vorzugsweise organische Basen, wie tertiäre Amine, beispielsweise Triethylamin, oder Pyridin, ferner anorganische Basen, wie beispielsweise Alkalihydroxide und Alkalicarbonate.

Die Reaktionstemperaturen können bei der Durchführung des erfindungsgemäßen Verfahrens (a) in einem größeren Bereich variiert werden. Im allgemeinen arbeitet man zwischen 0°C und 120°C, vorzugsweise zwischen 20°C und 100°C.

Bei der Durchführung des erfindungsgemäßen Verfahrens (a) setzt man vorzugsweise auf 1 Mol der Verbindung der Formel (II) 1 bis 1,5 Mol Halogenacetylierungsmittel und 1 bis 1,5 Mol Säurebinder ein. Die Isolierung der Verbindungen der Formel (I) erfolgt in üblicher Weise.

Als Verdünnungsmittel kommen für die erfindungsgemäße Umsetzung gemäß Verfahren (b) alle inerten, mit Wasser nicht mischbaren, organischen Lösungsmittel infrage. Hierzu gehören vorzugsweise Ether, wie Diethylether; aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Die erfindungsgemäße Umsetzung gemäß Verfahren (b) wird in Gegenwart eines Säurebindemittels durchgeführt. Als solche können alle üblichen Säurebindemittel verwendet werden. Hierzu gehören vorzugsweise anorganische Basen, wie beispielsweise Alkalihydroxide und Alkalicarbonate.

Die Reaktionstemperaturen können bei der Durchführung des erfindungsgemäßen Verfahrens (b) in einem größeren Bereich variiert werden. Im allgemeinen arbeitet man zwischen -70°C und +100°C, vorzugsweise zwischen -20°C und +80°C.

Bei der Durchführung des erfindungsgemäßen Verfahrens (b) setzt man vorzugsweise auf 1 Mol Halogenacetanilid der Formel (IV) 1 bis 1,5 Mol Diazolyl-alkyl-Derivat der Formel (V) ein. Die Isolierung der Verbindungen der Formel (I) erfolgt in üblicher Weise.

In einer bevorzugten Ausführungsform wird die erfindungsgemäße Umsetzung gemäß Verfahren (b) in einem Zweiphasensystem, wie beispielsweise wässrige Natron- oder Kalilauge/Toluol oder Methylenchlorid, gegebenenfalls unter Zusatz von 0,1-1 Mol eines Phasen-Transfer-Katalysators, wie beispielsweise Ammonium- oder Phosphoniumverbindungen, beispielsweise seien Benzyl-dodecyl-dimethyl-ammoniumchlorid (Zephirol) und Triethyl-benzyl-ammoniumchlorid genannt, durchgeführt (vergleiche auch die Herstellungsbeispiele).

Sowohl die nach dem Verfahren (a) als auch die nach dem Verfahren (b) herstellbaren erfindungsgemäßen Stoffe der Formel (I) können in Säureadditions-Salze bzw. Metallsalz-Komplexe überführt werden.

Zur Herstellung von physiologisch verträglichen Säureadditionssalzen der Verbindungen der Formel (I) kommen vorzugsweise folgende Säuren in Frage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure,Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe des Periodensystems in Frage, wobei Kupfer, Zink, Mangan, Magnesium, Zinn, Eisen und Nickel beispielhaft genannt seien.

0019742



Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol, und Hinzufügen dieser Lösung zur Verbindung der Formel (I). Man kann Metallsalz-Komplexe in bekannter Weise, z.B. durch Abfiltrieren, Isolieren und gegebenenfalls durch Umkristallisation reinigen.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen insbesondere neben einer sehr guten Wirkung gegen Gräser und gegen Cyperus-Arten auch eine gute herbizide Wirkung bei breitblättrigen Unkräutern. Hervorzuheben sind die selektiven Einsatzmöglichkeiten der erfindungsgemäßen Wirkstoffe vorzugsweise in Mais, Zuckerrüben, Sojabohnen und Baumwolle.

In bestimmten Aufwandmengen zeigen die erfindungsgemäßen Stoffe auch eine wachstumsregulierende Wirkung, wie insbesondere eine Wuchshemmung; sowie eine fungizide Wirkung, insbesondere gegen Oomyceten und Pyricularia oryzae bei Reis.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzol, Chlorethylen oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser.
Als feste Trägerstoffe kommen in Frage: z.B. natürliche Gesteinsmehle, wie Kaoline, Tonerden, Talkum, Kreide, Quarz, Attapilgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der

Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,1 und 10 kg Wirkstoff pro ha, vorzugsweise zwischen 0,25 und 5 kg/ha.

Wirkung und Einsatzmöglichkeiten zur Unkrautbekämpfung gehen aus dem Beispiel A hervor.

In dem nachfolgenden Beispiel wird die nachstehend angegebene Verbindung als Vergleichssubstanz eingesetzt:

A =

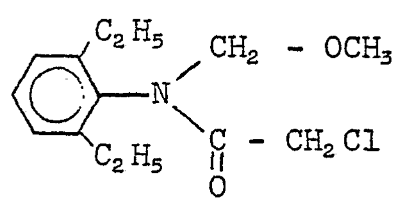

(2,6-Diethyl)-N-methoxymethyl-chloracetanilid

Beispiel A

Pre-emergence-Test

Lösungsmittel: 5 Gewichtsteile Aceton

Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 %=keine Wirkung (wie unbehandelte Kontrolle)

100 %=totale Vernichtung

Die erfindungsgemäßen Wirkstoffe 1,3,4,7 und 8 zeigen in diesem Test eine bessere selektiv herbizide Wirksamkeit als die aus dem Stand der Technik bekannte Substanz (A).

## Herstellungsbeispiele

### Beispiel 1

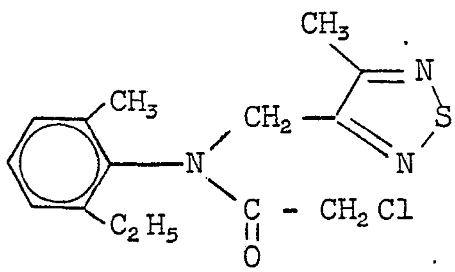

(Verfahren a)

59g (0,239 Mol) 2-Ethyl-6-methyl-N-(4-methyl-1,2,5-thiadiazol-3-yl-methyl)anilin und 20g (0,25 Mol) Pyridin werden in 200 ml absolutem Tetrahydrofuran gelöst und unter Rühren tropfenweise mit 28,25g (0,25 Mol) Chloracetylchlorid versetzt. Die Innentemperatur steigt dabei auf 60°C an. Es wird noch 1 Stunde bei Raumtemperatur nachgerührt. Danach wird die Reaktionslösung eingeengt und mit Wasser versetzt. Das kristalline Reaktionsprodukt wird abgesaugt und mit Wasser gewaschen. Nach dem Trocknen erhält man 70,5g (91 % der Theorie) 2-Ethyl-6-methyl-N-(4-methyl-1,2,5-thiadiazol-3-yl-methyl)-chloracetanilid als beigefarbenes Pulver vom Schmelzpunkt 89-93°C.

### Herstellung der Vorstufen

(II-1) 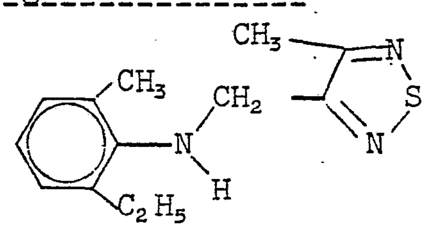

Ein Gemisch aus 68g (0,652 Mol) 2-Ethyl-6-methyl-anilin, 45g (0,326 Mol) gepulvertem Kaliumcarbonat und 30 ml Dimethylformamid wird auf 100°C erhitzt und unter Rühren tropfenweise mit 63g (0,326 Mol) 3-Brommethyl-4-methyl-1,2,5-thiadiazol versetzt. Es wird noch 5 Stunden

bei 100°C gerührt, vom anorganischen Niederschlag abfiltriert und das Filtrat destilliert. Man erhält 59,4g (77,4 % der Theorie) 2-Ethyl-6-methyl-N-(4-methyl-1,2,5-thiadiazol-3-yl-methyl)-anilin vom Siedepunkt 160-163°C/0,3 Torr.

(V-1) 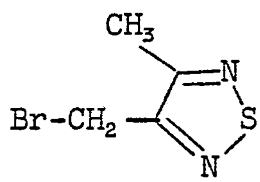

30g (0,263 Mol) 3,4-Dimethyl-1,2,5-thiadiazol und 46,8g (0,263 Mol) 4-Bromsuccinimid werden unter Zusatz von 100 mg Azodiisobutyronitril in 200 ml absolutem Tetrachlorkohlenstoff unter Rückfluß erhitzt. Anschließend wird das gebildete Succinimid abfiltriert und das Filtrat destilliert. Man erhält 25,3g (50% der Theorie) 3-Brommethyl-4-methyl-1,2,5-thiadiazol vom Siedepunkt 50-56°C/0,1 Torr.

Beispiel 2

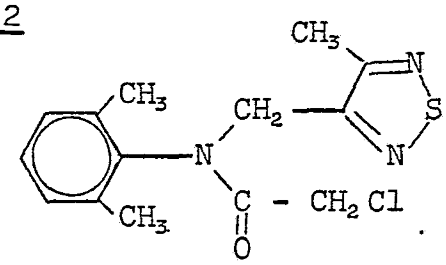

(Verfahren b)

Zu einem schnell gerührten 2-Phasen-Gemisch aus 4,5g (0,022 Mol) 2,6-Dimethylchloracetanilid, 200 ml Triethylbenzylammoniumchlorid, 25 ml 50 %-iger Natronlauge und 100 ml Methylenchlorid werden bei Raumtemperatur 5,2g 3-Brommethyl-4-methyl-1,2,5-thiadiazol schnell zugetropft. Nach 1 Stunde Rühren wird die organische Phase abgetrennt, mehrmals mit Wasser gewaschen,

über Natriumsulfat getrocknet und eingeeengt. Das kristalline Rohprodukt wird aus Petrolether umkristallisiert. Man erhält 3,5g (51 % der Theorie) 2,6-Dimethyl-N-(4-methyl-1,2,5-thiadiazol-3-yl-methyl)-chloracetanilid vom Schmelzpunkt 84-85°C.

Beispiel 3

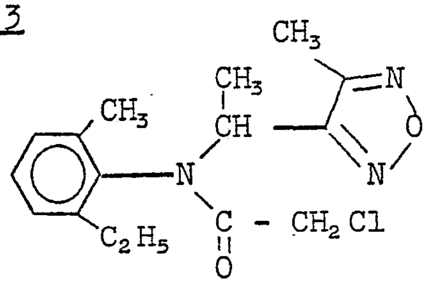

(Verfahren a)

14,5g (0,06 Mol) 2-Ethyl-6-methyl-N-(4-methyl-1,2,5-oxadiazol-3-yl-eth-1-yl)-anilin und 4,75g (0,06 Mol) Pyridin werden in 100 ml absolutem Tetrahydrofuran zum Sieden erhitzt und unter Rühren tropfenweise mit 6,8g (0,06 Mol) Chloracetylchlorid versetzt. Nach beendeter Zugabe läßt man 5 Minuten unter Rückfluß rühren, engt das Reaktionsgemisch ein, nimmt den Rückstand in Methylenchlorid auf und schüttelt mit Wasser aus. Die organische Phase wird über Natriumsulfat getrocknet und eingeengt. Nach Reinigung des Rückstandes über eine Kieselgelsäule und Umkristallisation aus Petrolether erhält man 9,6g (49,8 % der Theorie) 2-Ethyl-6-methyl-N-(4-methyl-1,2,5-oxadiazol-3-yl-eth-1-yl)-chloracetanilid vom Schmelzpunkt 91-92°C.

Herstellung der Vorstufen

(II-2)

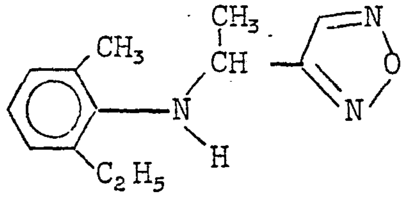

Enstsprechend Beispiel 1 erhält man aus 10,8 g (0,08 Mol) 2-Ethyl-6-methyl-anilin, 11,0 g (0,08 Mol) gepulvertem Kaliumcarbonat und 15,3g (0,08 Mol) 3-(1-Brom-eth-1-yl)-4-methyl-1,2,5-oxadiazol 14,8g (75,5 % der Theorie) 2-Ethyl-6-methyl-N-(4-methyl-1,2,5-oxadiazol-3-yl-eth-1-yl)-anilin, das direkt weiter umgesetzt wird.

(V-2) 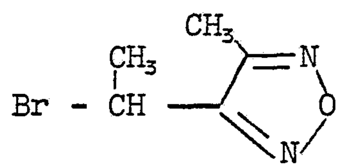

Entsprechend Beispiel 1 erhält man aus 13,5g (0,12 Mol) 3-Ethyl-4-methyl-1,2,5-oxadiazol und 21,4g (0,12 Mol) N-Bromsuccinimid unter Zusatz von 0,2 g Azodiisobutyronitril in 150 ml absolutem Tetrachlorkohlenstoff 14,1g (61,5 % der Theorie) 3-(1-Brom-eth-1-yl)-4-methyl-1,2,5-oxadiazol vom Siedepunkt 78°C/12 Torr.

In entsprechender Weise werden diejenigen Verbindungen erhalten, die in der Tabelle 4 formelmäßig aufgeführt sind.

Tabelle 4

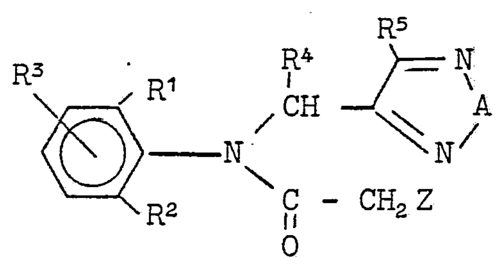 (I)

| Bsp. Nr. | $R^1$ | $R^2$ | $R^3$ | $R^4$ | $R^5$ | A | Z | Schmelzpunkt(°C) |
|---|---|---|---|---|---|---|---|---|
| 4 | $C_2H_5$ | $C_2H_5$ | H | H | $CH_3$ | S | Cl | Oel |
| 5 | $CH_3$ | $CH_3$ | H | $CH_3$ | $CH_3$ | O | Cl | 124-26 |
| 6 | $C_2H_5$ | $C_2H_5$ | H | $CH_3$ | $CH_3$ | O | Cl | 72 |
| 7 | $CH_3$ | $C_2H_5$ | H | H | $CH_3$ | O | Cl | Oel |
| 8 | $CH_3$ | $CH_3$ | H | H | $CH_3$ | O | Cl | 79-80 |
| 9 | $C_2H_5$ | $C_2H_5$ | H | H | $CH_3$ | O | Cl | 80-82 |
| 10 | $CH_3$ | $CH_3$ | H | $C_2H_5$ | $CH_3$ | O | Cl | 113 |

Nach dem in der Anmeldung beschriebenen Verfahren und entsprechend den Beispielen 1 und 3 werden die in der nachstehenden Tabelle 5 formelmäßig aufgeführten Ausgangsprodukte der Formel (II) erhalten:

Tabelle 5

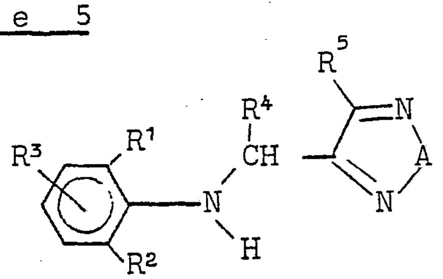

(II)

| Bsp. Nr. | $R^1$ | $R^2$ | $R^3$ | $R^4$ | $R^5$ | A | Physikalische Konstante |
|---|---|---|---|---|---|---|---|
| (II-3) | $CH_3$ | $CH_3$ | H | H | $CH_3$ | S | nicht isoliert |
| (II-4) | $C_2H_5$ | $C_2H_5$ | H | H | $CH_3$ | S | Kp.130-40°C/0,1 |
| (II-5) | $CH_3$ | $CH_3$ | H | $CH_3$ | $CH_3$ | O | nicht isoliert |
| (II-6) | $C_2H_5$ | $C_2H_5$ | H | $CH_3$ | $CH_3$ | O | nicht isoliert |
| (II-7) | $CH_3$ | $C_2H_5$ | H | H | $CH_3$ | O | nicht isoliert |
| (II-8) | $CH_3$ | $CH_3$ | H | H | $CH_3$ | O | nicht isoliert |
| (II-9) | $C_2H_5$ | $C_2H_5$ | H | H | $CH_3$ | O | nicht isoliert |

Patentansprüche:

1) N-(2,5-Diazolyl)-alkyl-halogenacetanilide der Formel

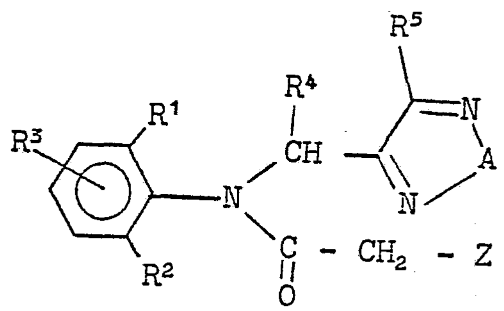

(I)

in welcher

A für Sauerstoff, Schwefel oder die Gruppierung >N-R steht, worin

R für Alkyl steht,

$R^1$ für Wasserstoff, Alkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wassertoff oder Alkyl steht,

$R^5$ für Wasserstoff, Alkyl, Alkoxy, Alkylthio, Dialkylamino, Halogen, Cyano oder Alkoxycarbonyl steht und,

Z für Halogen steht,

sowie deren Säureadditions-Salze und Metallsalz-Komplexe.

2) Verfahren zur Herstellung von N-(2,5-Diazolyl)alkyl-halogenacetaniliden der Formel (I) sowie von deren Säureadditions-Salzen und Metallsalz-Komplexen, dadurch gekennzeichnet, daß man

a) N-(2,5-Diazolyl)alkyl-aniline der Formel

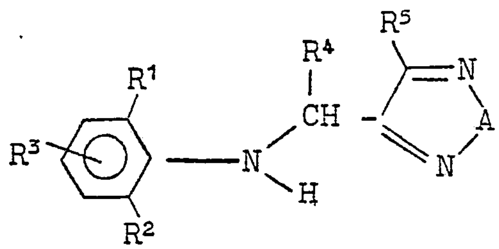

(II)

in welcher
A, $R^1$, $R^2$, $R^3$, $R^4$ und $R^5$ die oben angegebene Bedeutung haben,

mit Halogenessigsäurechloriden oder- bromiden bzw.- anhydriden der Formeln

$$Z - CH_2 - CO - Cl(Br) \qquad (IIIa)$$

bzw.

$$(Z - CH_2 - CO)_2O \qquad (IIIb)$$

in welchen

Z die oben angegebene Bedeutung hat,

in Gegenwart eines Verdünnungsmittels und gegebenenfalls in Gegenwart eines Säurebindemittels umsetzt,

oder

b) Halogenacetanilide der Formel

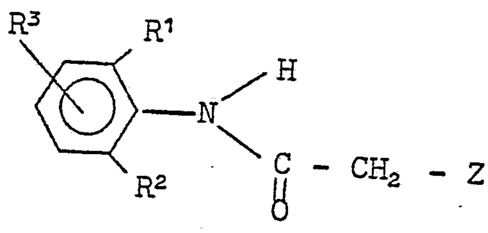

(IV)

in welcher

$R^1$, $R^2$, $R^3$ und Z die oben angegebene Bedeutung haben,

mit Diazolylalkyl-Derivaten der Formel

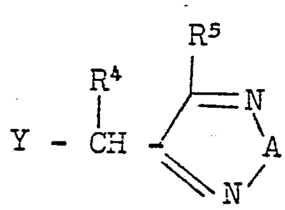

(V)

in welcher

A, $R^4$ und $R^5$ die oben angegebene Bedeutung haben und

Y für Halogen, dem Mesyl- oder Tosyl-Rest steht,

in Gegenwart eines Säurebinders und gegebenenfalls in Gegenwart eines organischen Verdünnungsmittels, oder in einem wässrig- organischen Zweiphasensystem in Gegenwart eines Phasentransfer-Katalysators umsetzt,

und gegebenenfalls anschließend eine Säure oder ein Metallsalz addiert.

3) Herbizide Mittel, gekennzeichnet durch einen Gehalt an mindestens einem N-(2,5-Diazolyl)-alkyl-halogenacetanilid der Formel (I) bzw. eines Säureadditions-Salzes oder eines Metallsalz-Komplexes eines N-(2,5-Diazolyl)-alkyl-halogenacetanilides der Formel (I).

4) Verfahren zur Bekämpfung von Unkräutern, dadurch gekennzeichnet, daß man N-(2,5-Diazolyl)-alkyl-halogenacetanilide der Formel (I) bzw. Säureadditions-Salze oder Metallsalz-Komplexe von N-(2,5-Diazolyl)-alkyl-halogenacetaniliden der Formel (I) auf die Unkräuter und/oder deren Lebensraum ausbringt.

5) Verwendung von N-(2,5-Diazolyl)-alkyl-halogenacetaniliden der Formel (I) bzw. von Säureadditions-Salzen oder Metallsalz-Komplexen von N-(2,5-Diazolyl)-alkyl-halogenacetaniliden der Formel (I) zur Bekämpfung von Unkräutern.

6) Verfahren zur Herstellung herbizider Mittel, dadurch gekennzeichnet, daß man N-(2,5-Diazolyl)-alkyl-halogenacetanilide der Formel (I) bzw. Säureadditions-Salze oder Metallsalz-Komplexe von N-(2,5-Diazolyl)-alkyl-halogenacetaniliden der Formel (I) mit Streckmitteln und/oder oberflächenaktiven Stoffen vermischt.

7) N-(2,5-Diazolyl)-alkyl-aniline der Formel

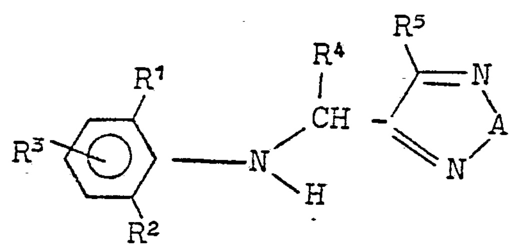

(II)

in welcher

A für Sauerstoff, Schwefel oder die Gruppierung >N-R steht, worin

R für Alkyl steht,

$R^1$ für Wasserstoff, ALkyl oder Alkoxy steht,

$R^2$ für Wasserstoff, Alkyl, Alkoxy oder Halogen steht,

$R^3$ für Wasserstoff, Alkyl, Alkoxy oder Halogen steht,

$R^4$ für Wasserstoff oder Alkyl steht und

$R^5$ für Wasserstoff, Alkyl, Alkoxy, Alkylthio, Dialkylamino, Halogen, Cyano oder Alkoxycarbonyl steht.

8) Verfahren zur Herstellung von N-(2,5-Diazolyl)-alkylanilinen der Formel (II), dadurch gekennzeichnet, daß man Aniline der Formel

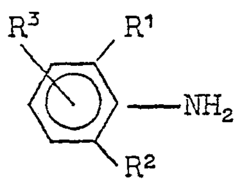

(VI)

in welcher

$R^1$, $R^2$ und $R^3$ die oben angegebene Bedeutung haben,

mit Diazolylalkyl-Derivaten der Formel

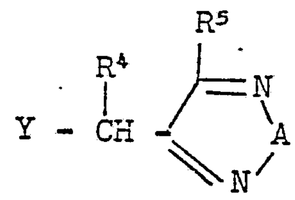

(V)

in welcher

A, $R^4$, $R^5$ und Y die oben angegebene Bedeutung haben,

in Gegenwart eines Säurebinders und gegebenenfalls in Gegenwart eines Verdünnungsmittels umsetzt.

9) Verbindung der Formel

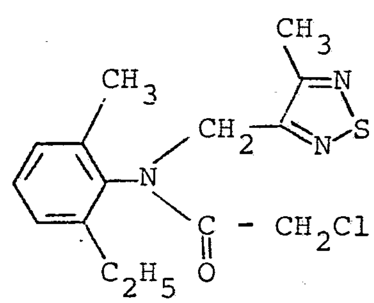

10) Verbindung der Formel